(19) Europäisches Patentamt
European Patent Office
Office européen des brevets

(11) Veröffentlichungsnummer : **0 084 597**
**B1**

(12) # EUROPÄISCHE PATENTSCHRIFT

(45) Veröffentlichungstag der Patentschrift : **02.01.86**

(21) Anmeldenummer : **82108908.3**

(22) Anmeldetag : **27.09.82**

(51) Int. Cl.[4] : **C 07 D249/08**, C 07 D233/60, A 61 K 31/41, A 61 K 31/415

(54) **Triazolyl- und Imidazolderivate sowie diese enthaltende pharmazeutische Zubereitungen.**

(30) Priorität : **10.10.81 DE 3140277**

(43) Veröffentlichungstag der Anmeldung : **03.08.83 Patentblatt 83/31**

(45) Bekanntmachung des Hinweises auf die Patenterteilung : **02.01.86 Patentblatt 86/01**

(84) Benannte Vertragsstaaten : **AT BE CH DE FR GB IT LI NL SE**

(56) Entgegenhaltungen :
**EP-A- 0 007 505**
**EP-A- 0 048 548**
**DE-A- 2 708 987**
**DE-A- 2 820 489**

(73) Patentinhaber : **BAYER AG**
**Konzernverwaltung RP Patentabteilung**
**D-5090 Leverkusen 1 Bayerwerk (DE)**

(72) Erfinder : **Elbe, Hans-Ludwig, Dr.**
**Dasnöckel 59**
**D-5600 Wuppertal 11 (DE)**
Erfinder : **Bückel, Karl Heinz, Prof. Dr.**
**Dabringhausener Strasse 42**
**D-5093 Burscheid (DE)**
Erfinder : **Schaller, Klaus, Dr.**
**Bergerheide 47**
**D-5600 Wuppertal 1 (DE)**
Erfinder : **Plempel, Manfred, Dr.**
**Pahlkestrasse 5**
**D-5600 Wuppertal 1 (DE)**

**EP 0 084 597 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die vorliegende Erfindung betrifft die Verwendung von neuen Phenoxyphenyl-azolylmethyl-ketonen und -carbinolen bei der Behandlung von Krankheiten.

Es wurde gefunden, daß die neuen Phenoxyphenyl-azolylmethyl-ketone und -carbinole der allgemeinen Formel I

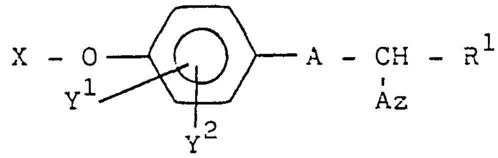

(I)

in welcher

Az für 1,2,4-Triazol-1-yl und -4-yl oder Imidazol-1-yl steht,

A für die Ketogruppe, die —CH(OH)— oder die —C(OH)R-Gruppierung steht,

R für Alkyl mit 1 bis 4, Alkenyl mit 2 bis 4 Kohlenstoffatomen oder gegebenenfalls durch Chlor substituiertes Benzyl steht,

$R^1$ für geradkettiges oder verzweigtes Alkyl mit 1 bis 8 Kohlenstoffatomen, Cycloalkyl und Cycloalkylmethyl mit jeweils 5 oder 6 Kohlenstoffatomen, Alkinyl mit 2 bis 4 Kohlenstoffatomen, Alkoxyalkyl und Alkylthioalkyl mit jeweils 1 bis Trifluormethyl, Trifluormethoxy, Trifluormethylthio, Methoxy, Methylthio, Amino, Dimethylamino, Methoxycarbonyl, Nitro oder Cyano in Frage kommen, steht ; und

$Y^1$ für Wasserstoff, Fluor, Chlor oder Methyl steht ;

$Y^2$ für Wasserstoff, Fluor oder Chlor steht ; und

X für gegebenenfalls ein- oder mehrfach durch Chlor oder Methyl oder einfach durch Brom substituiertes Phenyl steht,

sowie deren Säureadditionssalze gute pharmazeutische, insbesondere antimykotische Eigenschaften aufweisen.

Die Verbindungen der Formel (I) besitzen gegebenenfalls zwei asymmetrische Kohlenstoffatomen ; sie können in den beiden geometrischen Isomeren (threo- und erythro-Form) in verschiedenen Mengenverhältnissen vorliegen. In beiden Fällen liegen sie als optische Isomere vor.

Insbesondere genannt seien die Verbindungen der nachstehenden Formeln :

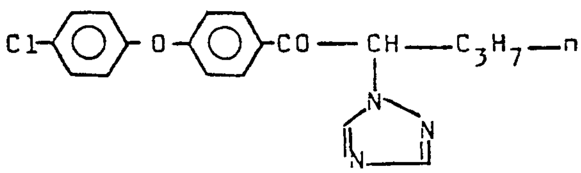

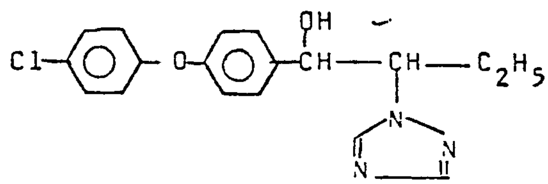

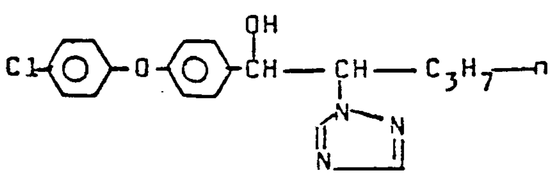

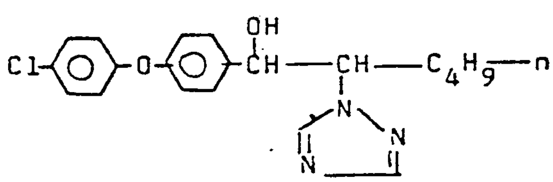

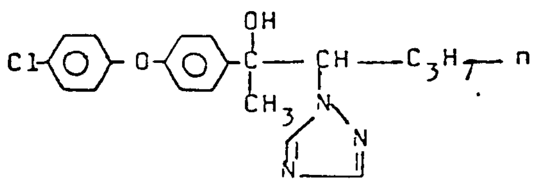

Aus der DE-A 27 08 987 sind bereits (1-Phenyl-2-triazolylethyl)-ether-derivate bekannt geworden, sie eignen sich als Mittel zur Regulierung des Pflanzenwachstums, nicht aber als antimykotische Mittel.

Fungizide und/oder antimykotische Imidazolyl- bzw. Triazolylderivate sind u. a. bekannt geworden aus EP-A 0 048 548, EP-A 0 008 505 und DE-A 2 820 489. All diese Verbindungen befriedigen jedoch nicht bezüglich ihrer antimykotischen Wirksamkeit und eignen sich daher nicht als Arzneimittel.

Die erfindungsgemäß zu verwendenden Wirkstoffe sind noch nicht bekannt. Sie können jedoch gemäß einem eigenen Vorschlag erhalten werden, indem man

a) Halogenketone der Formel

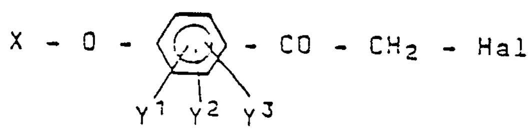

(II)

in welcher

Hal für Halogen, vorzugsweise Chlor oder Brom steht und

X, $Y^1$, $Y^2$ u. $Y^3$ die oben angegebene Bedeutung haben,

mit Azolen der Formel

$$H—Az \quad (III)$$

in welcher Az die oben angegebene Bedeutung hat, in Gegenwart eines inerten organischen Lösungsmittels, wie z. B. Acetonitril, und in Gegenwart eines Säurebindemittels, wie z. B. Kaliumcarbonat, bei Temperaturen von 60 bis 120 °C umsetzt, und gegebenenfalls

b) die so erhaltenen Azolylketone der Formel

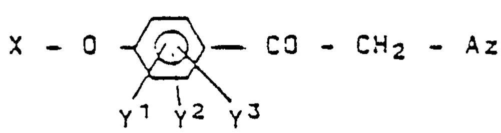

(Ia)

in welcher Az, X, $Y^1$, $Y^2$ und $Y^3$ die oben angegebene Bedeutung haben, mit einem Alkylierungsmittel der Formel

$$R^1—Z \quad (IV)$$

in welcher

$R^1$ die oben angegebene Bedeutung hat und

Z für eine elektronenanziehende Abgangsgruppierung steht,

in Gegenwart einer Base, wie z. B. Kaliumhydroxid, und in Gegenwart eines inerten organischen Lösungsmittels, wie z. B. Dimethylsulfoxid, bei Temperaturen von 20 bis 100 °C umsetzt, oder in einem wässig-organischen Zweiphasensystem, wie beispielsweise wässrige Natron- oder Kalilauge/Toluol oder Methylenchlorid, in Gegenwart eines Phasentransferkatalysators, wie beispielsweise Ammonium- oder Phosphoniumverbindungen, umsetzt ; und gegebenenfalls

c) die nach den Verfahren (a) und (b) erhaltenen Azolylketone der Formel

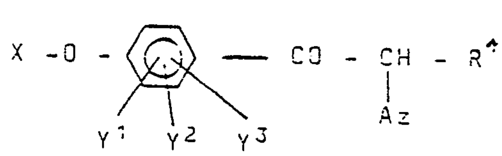

(Ib)

in welcher Az, X, $Y^1$, $Y^2$, $Y^3$ und $R^1$ die oben angegebene Bedeutung haben, in üblicher Weise reduziert, wie z. B. durch Umsetzung mit komplexen Hydriden, wie Natriumborhydrid oder Lithiumalanat, in Gegenwart eines polaren organischen Lösungsmittels, wie z. B. Alkohole, bei Temperaturen von ca. 0 bis 30 °C ; oder durch Umsetzung mit Aluminiumisopropylat in Gegenwart eines Verdünnungsmittels, wie z.

B. Isopropanol, bei Temperaturen von ca. 20 bis 120 °C ; oder durch Umsetzung mit metallorganischen Verbindungen der Formel

$$Me—R \quad (V)$$

in welcher

R die oben angegebene Bedeutung hat und

Me für ein Alkalimetall oder den Rest Hal'-Mg steht, wobei

Hal' für Chlor, Brom oder Iod steht,

in Gegenwart eines Verdünnungsmittels, wie wasserfreie Ether, bei Temperaturen von 0 bis 80 °C.

An die so erhaltenen Verbindungen der Formel (I) kann gegebenenfalls anschließend eine Säure oder ein Metallsalz addiert werden. In manchen Fällen erweist es sich als vorteilhaft, die Verbindungen der Formel (I) über ihre Salze in reiner Form zu erhalten.

Die Halogenketone der Formel (II) sind noch nicht bekannt, sie können jedoch in allgemein bekannter Art und Weise erhalten werden, indem man z. B. Diphenylether der Formel

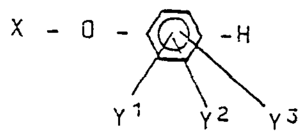

(VI)

in welcher X, $Y^1$, $Y^2$ und $Y^3$ die oben angegebene Bedeutung haben, mit Chlor(Brom) acetylchlorid(bromid) unter den Bedingungen einer Friedel-Crafts-Acylierung umsetzt (vergleiche auch die Herstellungsbeispiele).

Die Azole der Formel (III), die Alkylierungsmittel der Formel (IV) und die metallorganischen Verbindungen der Formel (V) sind allgemein bekannte Verbindungen der organischen Chemie.

Zur Herstellung von physiologisch verträglichen Säureadditionssalzen der Verbindungen der Formel (I) kommen vorzugsweise folgende Säuren infrage : Die Halogenwasserstoffsäuren, wie z. B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäuren und Hydroxycarbonsäuren, wie z. B. Essigsäure, Maleinsäure, Bernsteinsäure, Fumarsäure, Weinsäure, Zitronensäure, Salizylsäure, Sorbinsäure, Milchsäure, sowie Sulfonsäuren, wie z. B. p-Toluolsulfonsäure und 1,5-Naphthalindisulfonsäure. Die Säureadditionssalze der Verbindungen der Formel (I) können in einfacher Weise nach üblichen Salzbildungsmethoden, z. B. durch Lösen einer Verbindung der Formel (I) in einem geeigneten inerten Lösungsmittel und Hinzufügen der Säure, z. B. Chlorwasserstoffsäure, erhalten werden und in bekannter Weise, z. B. durch Abfiltrieren, isoliert und gegebenenfalls durch Waschen mit einem inerten organischen Lösungsmittel gereinigt werden.

Die erfindungsgemäß verwendbaren Verbindungen der Formel (I), ihre Säureadditionssalze weisen starke antimykotische Wirkungen auf. Sie besitzen ein sehr breites antimykotisches Wirkungsspektrum, insbesondere gegen Dermatophyten und Sproßpilze sowie bi-phasische Pilze, z. B. gegen Candida-Arten, wie Candida albicans, Epidermophyton-Arten, wie Epidermophyton floccosum, Aspergillus-Arten, wie Aspergillus niger und Aspergillus fumigatus, Trichophyton-Arten, wie Trichophyton mentagrophytes, Microsporon-Arten, wie Microsporon felineum sowie Penicillium-Arten, wie Penicillium commune. Die Aufzählung dieser Mikroorganismen stellt keinesfalls eine Beschränkung der bekämpfbaren Keime dar, sondern hat nur erläuternden Charakter.

Als Indikationsgebiete in der Humanmedizin können beispielsweise genannt werden :

Dermatomykosen und Systemmykosen durch Trichophyton mentagrophytes und andere Trichophytonarten, Mikrosporonarten, Epidermophyton floccosum, Sproßpilze und biphasische Pilze sowie Schimmelpilze hervorgerufen.

Als Indikationsgebiet in der Tiermedizin können beispielsweise aufgeführt werden :

Alle Dermatomykosen und Systemmykosen, insbesondere solche, die durch die obengenannten Erreger hervorgerufen werden.

Zur vorliegenden Erfindung gehören pharmazeutische Zubereitungen, die neben nicht toxischen, inerten pharmazeutisch geeigneten Trägerstoffen einen oder mehrere erfindungsgemäße Wirkstoffe enthalten oder die aus einem oder mehreren erfindungsgemäßen Wirkstoff bestehen sowie Verfahren zur Herstellung dieser Zubereitungen.

Zur vorliegenden Erfindung gehören auch pharmazeutische Zubereitungen in Dosierungseinheiten. Dies bedeutet, daß die Zubereitungen in Form einzelner Teile, z. B. Tabletten, Dragges, Kapsel, Pillen, Suppositorien und Ampullen vorliegen, deren Wirkstoffgehalt einem Bruchteil oder einem Vielfachen einer Einzeldosis entspricht. Die Dosierungseinheiten können z. B. 1, 2, 3 oder 4 Einzeldosen oder 1/2, 1/3 oder 1/4 einer Einzeldosis enthalten. Eine Einzeldosis enthält vorzugsweise die Menge Wirkstoff, die bei einer Applikation verabreicht wird und die gewöhnlich einer ganzen, einer halben oder einem Drittel oder einem Viertel einer Tagesdosis entspricht.

Unter nicht toxischen, inerten pharmazeutisch geeigneten Trägerstoffen sind feste, halbfeste oder flüssige Verdünnungsmittel, Füllstoffe und Formulierungshilfsmittel jeder Art zu verstehen.

Als bevorzugte pharmazeutische Zubereitungen seien Tabletten, Dragees, Kapseln, Pillen, Granulate, Suppositorien, Lösungen, Suspensionen und Emulsionen, Pasten, Salben, Gele, Cremes, Lotions, Puder und Sprays genannt.

Tabletten, Dragees, Kapseln, Pillen und Granulate können den oder die Wirkstoffe neben den üblichen Trägerstoffen enthalten, wie (a) Füll- und Streckmittel, z. B. Stärken, Milchzucker, Rohrzucker, Glukose, Mannit und Kieselsäure, (b) Bindemittel, z. B. Carboxymethylcellulose, Alginate, Gelatine, Polyvinylpyrrolidon, (c) Feuchthaltemittel, z. B. Glycerin, (d) Sprengmittel, z. B. Agar-Agar, Calciumcarbonat und Natriumbicarbonat, (e) Lösungsverzögerer, z. B. Paraffin und (f) Resorptionsbeschleuniger, z. B. quartenäre Ammoniumverbindungen, (g) Netzmittel, z. B. Cetylalkohol, Glycerinmonostearat, (h) Adsorptionsmittel, z. B. Kaolin und Bentonit und (i) Gleitmittel, z. B. Talkum, Calcium- und Magnesiumstearat und feste Polyäthylenglykole oder Gemische der unter (a) bis (i) aufgeführten Stoffe.

Den Tabletten, Dragees, Kapseln, Pillen und Granulate können mit den üblichen gegebenenfalls Opakisierungsmittel enthaltenden Überzügen und Hüllen versehen sein und auch so zusammengesetzt sein, daß sie den ober die Wirkstoffe nur oder bevorzugt in einem bestimmten Teil des Intestinaltraktes, gegebenenfalls verzögert abgeben, wobei als Einbettungsmassen z. B. Polymersubstanzen und Wachse verwendet werden können.

Der oder die Wirkstoffe können gegebenenfalls mit einem oder mehreren der oben angegebenen Trägerstoffen auch in mikroverkapselter Form vorliegen.

Suppositorien können neben dem oder den Wirkstoffen die üblichen wasserlöslichen oder wasserunlöslichen Trägerstoffe enthalten, z. B. Polyäthylenglykole, Fette, z. B. Kakaofett und höhere Ester (z. B. $C_{14}$-Alkohol mit $C_{16}$-Fettsäure) oder Gemische dieser Stoffe.

Salben, Pasten, Cremes und Gele können neben dem oder den Wirkstoffen die üblichen Trägerstoffe enthalten, z. B. tierische und pflanzliche Fette, Wachse, Paraffine, Stärke, Tragant, Cellulosederivate, Polyäthylenglykole, Silicone, Bentonite, Kieselsäure, Talkum und Zinkoxid oder Gemische dieser Stoffe.

Puder und Sprays können neben dem oder den Wirkstoffen die üblichen Trägerstoffe enthalten, z. B. Milchzucker, Talkum, Kieselsäure, Aluminiumhydroxid, Calciumsilikat und Polyamidpulver oder Gemische dieser Stoffe. Sprays können zusätzlich die üblichen Treibmittel z. B. Chlorfluorkohlenwasserstoffe enthalten.

Lösungen und Emulsionen können neben dem oder den Wirkstoffen die üblichen Trägerstoffe wie Lösungsmittel, Lösungsvermittler und Emulgatoren, z. B. Wasser, Äthylalkohol, Isopropylalkohol, Äthylcarbonat, Äthylacetat, Benzylalkohol, Benzylbenzoat, Propylenglykol, 1,3-Butylenglykol, Dimethylformamid, Oele, insbesondere Baumwollsaatöl, Erdnußöl, Meiskeimöl, Olivenöl, Ricinusöl und Sesamöl, Glycerin, Glycerinformal, Tetrahydrofurfurylalkohol, Polyäthylenglykole und Fettsäureester des Sorbitans oder Gemische dieser Stoffe enthalten.

Zur parenteralen Applikation können die Lösungen und Emulsionen auch in steriler und blutisotonischer Form vorliegen.

Suspensionen können neben dem oder den Wirkstoffen die üblichen Trägerstoffe wie flüssige Verdünnungsmittel, z. B. Wasser, Äthylalkohol, Propylenglykol, Suspendiermittel, z. B. äthoxylierte Isostearylalkohole, Polyoxyäthylensorbit- und Sorbitanester, mikrokristalline Cellulose, Aluminiummetahydroxid, Bentonit, Agar-Agar und Tragant oder Gemische dieser Stoffe enthalten.

Die genannten Formulierungsformen können auch Färbemittel, Konservierungsstoffe sowie geruchs- und geschmackverbessernde Zusätze, z. B. Pfefferminzöl und Eukalyptusöl und Süßmittel, z. B. Saccharin enthalten.

Die therapeutisch wirksamen Verbindungen sollen in den oben aufgeführten pharmazeutischen Zubereitungen vorzugsweise in einer Konzentration von etwa 0,1 bis 99,5, vorzugsweise von etwa 0,5 bis 95 Gew.-% der Gesamtmischung vorhanden sein.

Die oben aufgeführten pharmazeutischen Zubereitungen können außer den erfindungsgemäßen Wirkstoffen auch weitere pharmazeutische Wirkstoffe enthalten.

Die Herstellung der oben aufgeführten pharmazeutischen Zubereitungen erfolgt in üblicher Weise nach bekannten Methoden, z. B. durch Mischen des oder der Wirkstoffe mit dem oder den Trägerstoffen.

Zur vorliegenden Erfindung gehört auch die Verwendung der erfindungsgemäßen Wirkstoffe sowie von pharmazeutischen Zubereitungen, die einen oder mehrere erfindungsgemäße Wirkstoffe enthalten, in der Human- und Veterinärmedizin zur Verhütung, Besserung und/oder Heilung vorzugsweise der oben angeführten Erkrankungen.

Die Wirkstoffe oder die pharmazeutischen Zubereitungen können lokal, oral, parenteral, intraperitoneal und/oder rectal, vorzugsweise parenteral, insbesondere intravenös appliziert werden.

Im allgemeinen hat es sich sowohl in der Human- als auch in der Veterinärmedizin als vorteilhaft erwiesen, den oder die erfindungsgemäßen Wirkstoffe in Gesamtmengen von etwa 10 bis etwa 300, vorzugsweise 50 bis 200 mg/kg Körpergewicht je 24 Stunden, gegebenenfalls in Form mehrerer Einzelgaben zur Erzielung der gewünschten Ergebnisse zu verabreichen.

Es kann jedoch erforderlich sein, von den genannten Dosierungen abzuweichen und zwar in Abhängigkeit von der Art und dem Körpergewicht des zu behandelnden Objekts der Art und der Schwere der Erkrankung, der Art der Zubereitung und der Applikation des Arzneimittels sowie dem Zeitraum bzw. Intervall, innerhalb welchem die Verabreichung erfolgt. So kann es in einigen Fällen ausreichend sein, mit weniger als der oben genannten Menge Wirkstoff auszukommen, während in anderen Fällen die oben

angeführte Wirkstoffmenge überschritten werden muß. Die Festlegung der jeweils erforderlichen optimalen Dosierung und Applikationsart der Wirkstoffe kann durch jeden Fachmann aufgrund seines Fachwissens leicht erfolgen.

Herstellungsbeispiele

Beispiel 1

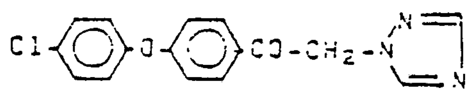

(Verfahren a)

100 g (0,36 Mol) ω-Chlor-4-(4'-chlorphenoxy)-acetophenon, 31,1 g (0,45 Mol) 1,2,4-Triazol und 124 g (0,9 Mol) gemahlenes Kaliumcarbonat werden 20 Stunden unter Rückfluß und Rühren erhitzt. Danach läßt man abkühlen, saugt die anorganischen Salze ab und engt das Filtrat ein. Der Rückstand wird in Methylenchlorid verrührt und mit verdünnter Salzsäure versetzt. Das entstehende Hydrochlorid wird abgetrennt, mit Methylenchlorid gewaschen und in üblicher Weise in die freie Base überführt. Man erhält 41,5 g (36,8 % der Theorie) 4-(4'-Chlorphenoxy)-ω-(1,2,4-triazol-1-yl)-acetophenon vom Schmelzpunkt 148-150 °C.

Herstellung der Ausgangsproduktes

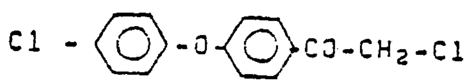

Zu 150 g (0,73 Mol) 4-Chlorbiphenylether und 117 g (0,88 Mol) Aluminiumtrichlorid in 1 000 ml Methylenchlorid läßt man bei Raumtemperatur 86,6 g (0,77 Mol) Chloracetylchlorid zutropfen. Die Reaktionslösung wird 1 Stunde nachgerührt und dann auf 3 l Eiswasser gegeben. Die organischen Phase wird abgetrennt, mit Wasser gewaschen, über Natriumsulfat getrocknet und eingeengt. Der Rückstand wird mit Petrolether verrieben, abgesaugt und getrocknet. Man erhält 168,7 g (82,3 % der Theorie) -Chlor-4-(4'-chlorphenoxy)-acetophenon vom Schmelzpunkt 59-61 °C.

Beispiel 2

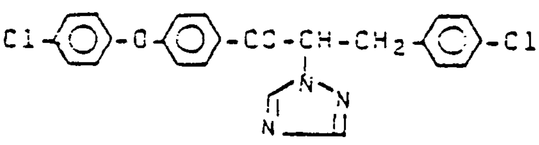

(Verfahren b)

31,4 g (0,1 Mol) 4-(4'-Chlorphenoxy)-ω-(1,2,4-triazol-1-yl)-acetophenon (Beispiel 1), 16,1 g 4-Chlorbenzylchlorid und 5,6 g (0,1 Mol) Kaliumhydroxid werden in 200 ml Dimethylsulfoxid 20 Stunden bei 40 °C gerührt. Anschließend wird das Reaktionsgemisch auf 700 ml Wasser gegeben und mit Essigester extrahiert. Die organische Phase wird mit Wasser gewaschen, über Natriumsulfat getrocknet und eingeengt. Der Rückstand wird zur Reinigung mit Isopropylether ausgekocht. Man erhält 26,1 g (59,6 % der Theorie) 1-(4-Chlorphenyl(-3-[4-(4'-chlorphenoxy)-phenyl]-2-(1,2,4-triazol-1-yl)-propan-3-on vom Schmelzpunkt 132 °C.

Beispiel 3

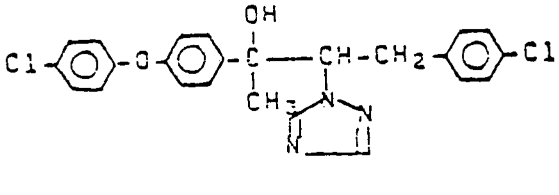

(Verfahren c)

7 g (0,016 Mol) 1-(4-Chlorphenyl)-3-[4-(4'-chlorphenoxy)-phenyl]-2-(1,2,4-triazol-1-yl)-propan-3-on

(Beispiel 2) und 4,8 g (0,04 Mol) Methylmagnesiumbromid, gelöst in 50 ml Ether, werden in 100 ml Tetrahydrofuran 1 Stunde unter Rückfluß gerührt. Man läßt abkühlen und versetzt mit verdünnter Salzsäure. Die organische Phase wird abgetrennt, mit Wasser gewaschen, über Natriumsulfat getrocknet und eingeengt. Der Rückstand wird in Diisopropylether verrührt, abgesaugt und getrocknet. Man erhält 5,3 g (73 % der Theorie) 1-(4-Chlorphenyl)-3-[4-(4'-chlorphenoxy)-phenyl]-2-(1,2,4-triazol-1-yl)-butan-3-ol vom Schmelzpunkt 159 °C.

Beispiel 4

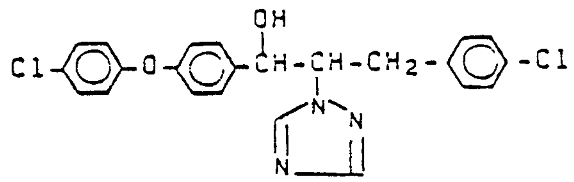

(Verfahren c)

14 g (0,032 Mol) 1-(4-Chlorphenoxy)-3-[4-(4'-chlorphenoxy)-phenyl]-2-(1,2,4-triazol-1-yl)-propan-3-on (Beispiel 2) in 400 ml Methanol werden mit 0,38 g (0,01 Mol) Natriumborhydrid, gelöst in 10 ml Wasser, versetzt. Man läßt 2 Stunden bei 25 °C rühren und stellt dann durch Zugabe von verdünnter Salzsäure auf einen pH-Wert von 6-7 ein. Die Reaktionslösung wird eingeengt und der Rückstand mit Methylenchlorid/Wasser aufgenommen. Man trennt die organische Phase ab, trocknet über Natriumsulfat und engt ein. Der feste Rückstand wird aus Diisopropylether umkristallisiert. Man erhält 10,5 g (75 % der Theorie) 1-(4-Chlorphenyl)-3-[4-(4'-chlorphenoxy)-phenyl]-2-(1,2,4-triazol-1-yl)-propan-3-ol vom Schmelzpunkt 112 °C.

In analoger Weise und entsprechend den erfindungsgemäßen Verfahren werden die in der nachfolgenden Tabelle aufgeführten Verbindungen der allgemeinen Formel (IA)

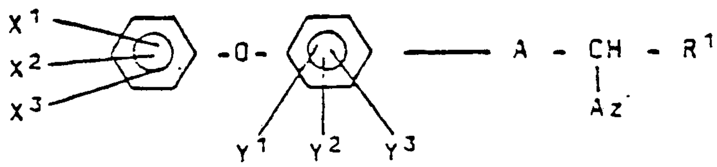

(IA)

| Bsp. Nr. | $X^1$ | $X^2$ | $X^3$ | $Y^1$ | $Y^2$ | $Y^3$ | A | Az | $R^1$ | Fp(°C) bzw. $n_D^{20}$ |
|---|---|---|---|---|---|---|---|---|---|---|
| 5 | H | H | H | H | H | H | -CO- | 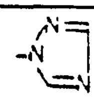 | $-C_3H_7$-n | 66-68 |
| 6 | 4-Cl | H | H | H | H | H | -CO- | 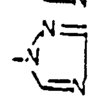 | $-C_3H_7$-n | 64-66 |
| 7 | 4-Cl | H | H | H | H | H | -CO- | 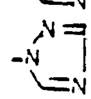 | $-C_2H_5$ | 91-92 |
| 8 | H | H | H | H | H | H | -CO- | 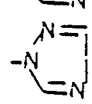 | $-C_2H_5$ | 62-63 |
| 9 | 4-Cl | H | H | H | H | H | -CO- | 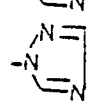 | $-CH_3$ | 1,6061 |
| 10 | 4-Cl | H | H | H | H | H | -CO- | 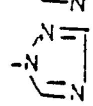 | $-C_4H_9$-n | 88 |
| 11 | 4-Cl | H | H | H | H | H | -CO- | 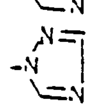 | 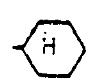 | 126 |
| 12 | 4-Cl | H | H | H | H | H | -CO- | 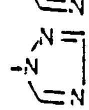 | $-C_5H_{11}$-n | 64 |

(Fortsetzung)

| Bsp. Nr. | $X^1$ | $X^2$ | $X^3$ | $Y^1$ | $Y^2$ | $Y^3$ | A | Az | $R^1$ | Fp(°C)bzw. $n_D^{20}$ |
|---|---|---|---|---|---|---|---|---|---|---|
| 13 | 4-Cl | H | H | H | H | H | -CO- | 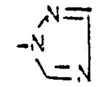 | $-C_3H_7$-i | 92-93 |
| 14 | 4-Cl | H | H | H | H | H | -CO- | 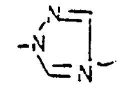 | $-CH_2C{\equiv}CH$ | 116 |
| 15 | 4-Cl | H | H | H | H | H | -CO- | 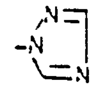 | $-CH_2CH{=}CH_2$ | 87 |
| 16 | 4-Cl | H | H | H | H | H | -CO- | 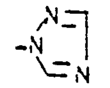 | $-C_7H_{15}$-n | 67 |
| 17 | 4-Cl | H | H | H | H | H | -CO- | 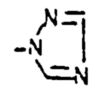 | $-CH_2CH{=}CHCH_3$ | 87 |
| 18 | 4-Cl | H | H | H | H | H | -CO- | 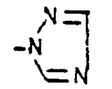 | $-CH_2CH_2OCH_3$ | 1,5940 |
| 19 | 4-Cl | H | H | H | H | H | -CO- | 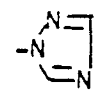 | $-CH{=}CHC_3H_7$ | 1,5840 |
| 20 | 2-Cl | 4-Cl | H | H | H | H | -CO- | 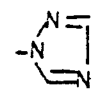 | $-C_3H_7$-n | 106 |
| 21 | 4-Cl | H | H | H | H | H | -CO- | 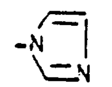 | $-C_2H_5$ | 1,6092 |
| 22 | 4-Cl | H | H | H | H | H | -CO | 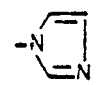 | $-C_3H_7$-n | 1,6040 |
| 23 | 4-Cl | H | H | H | H | H | -CO- | 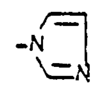 | $-CH_3$ | 1,5990 |
| 24 | 4-Cl | H | H | H | H | H | -CO- | 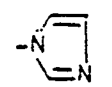 | $-C_4H_9$-n | 1,5959 |
| 25 | 4-Cl | H | H | H | H | H | -CO | 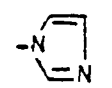 | $-C_3H_7$-i | 114-116 |
| 26 | 4-Cl | H | H | H | H | H | -CH(OH)- | 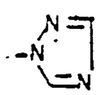 | $-C_2H_5$ | Oel(A-Form) |
| 27 | 4-Cl | H | H | H | H | H | -CH(OH)- | 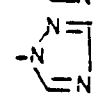 | $-C_2H_5$ | 161-62 (B-Form) |
| 28 | 4-Cl | H | H | H | H | H | -CH(OH)- | 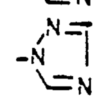 | $-C_3H_7$-n | 150(A-Form) |
| 29 | 4-Cl | H | H | H | H | H | -CH(OH)- | 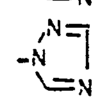 | $-C_3H_7$-n | 106-07 (B-Form) |
| 30 | 4-Cl | H | H | H | H | H | -CH(OH)- | 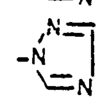 | $-C_4H_9$-n | 90(A-Form) |

A- und B-Form = die beiden möglichen geometrischen Isomeren

(Fortsetzung)

| Bsp. Nr. | $X^1$ | $X^2$ | $X^3$ | $Y^1$ | $Y^2$ | $Y^3$ | A | Az | $R^1$ | Fp(°C) bzw. $n_D^{20}$ |
|---|---|---|---|---|---|---|---|---|---|---|
| 31 | 4-Cl | H | H | H | H | H | -CH(OH)- | 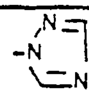 | $-C_4H_9-n$ | 116 (B-Form) |
| 32 | 4-CL | H | H | H | H | H | -CH(OH)- | 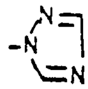 | $-CH_3$ | 90-100 |
| 33 | 4-Cl | H | H | H | H | H | -CH(OH)- | 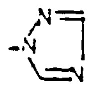 | $-C_7H_{15}-n$ | 74 |
| 34 | 4-Cl | H | H | H | H | H | -CH(OH)- | 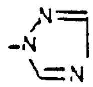 | $-CH_2CH=CHCH_3$ | 109 |
| 35 | 4-Cl | H | H | H | H | H | -CH(OH)- | 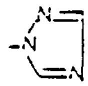 | $-CH=CHC_3H_7$ | 1,5603 |
| 36 | 4-Cl | H | H | H | H | H | -CH(OH)- | 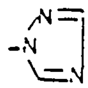 | $-CH_2CH_2OCH_3$ | 95-100 |
| 37 | 2-Cl | 4-Cl | H | H | H | H | -CH(OH)- | 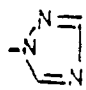 | $-C_3H_7-n$ | zähes Oel |
| 38 | 2-Cl | 4-Cl | H | H | H | H | -CH(OH)- | 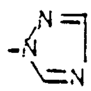 | $-CH_3$ | 200(xHCl) |
| 39 | 4-Cl | H | H | H | H | H | -C (OH)<br>$CH_2$- 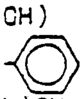 | 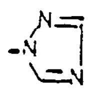 | $-C_3H_7-n$ | Oel |
| 40 | 4-Cl | H | H | H | H | H | $-C(OH)CH_3$ | 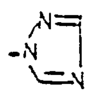 | $-C_3H_7-n$ | 145 |
| 41 | 4-Cl | H | H | H | H | H | $-C(OH)CH_3$ | 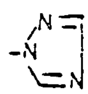 | $-CH_3$ | 128 |
| 42 | 4-Cl | H | H | H | H | H | -C(OH)<br>$CH_2$- 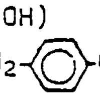 -Cl | 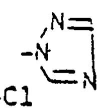 | $-CH_3$ | 142 |
| 43 | 4-Cl | H | H | H | H | H | -C(OH)<br>$CH_2-CH=CH_2$ | 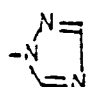 | $-C_3H_7-n$ | 128 |
| 44 | H | H | H | H | H | H | -CH(OH)- | 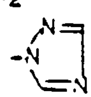 | $-CH_3$ | 1,5842 |
| 45 | H | H | H | H | H | H | -CH(OH)- | 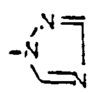 | $-C_3H_7-n$ | 96(A-Form) |
| 46 | H | H | H | H | H | H | -CH(OH)- | 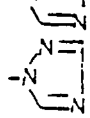 | $-C_3H_7-n$ | 102 (B-Form) |
| 47 | H | H | H | H | H | H | -CH(OH)- | 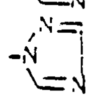 | $-C_3H_7-i$ | 114-117 |
| 48 | 4-Cl | H | H | H | H | H | -CH(OH)- | 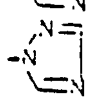 | 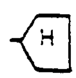 | 158 |

A- und B-Form = die beiden möglichen geometrischen Isomeren

(Fortsetzung)

| Bsp. Nr. | $X^1$ | $X^2$ | $X^3$ | $Y^1$ | $Y^2$ | $Y^3$ | A | Az | $R^1$ | Fp(°C)bzw. $n_D^{20}$ |
|---|---|---|---|---|---|---|---|---|---|---|
| 49 | H | H | H | H | H | H | -CH(OH)- | 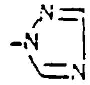 | $-C_2H_5$ | 121 (A-Form) |
| 50 | H | H | H | H | H | H | -CH(OH)- | 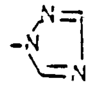 | $-C_2H_5$ | 120 (B-Form) |
| 51 | 4-Cl | H | H | H | H | H | -CH(OH)- | 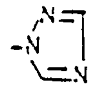 | $-C_5H_{11}$-n | 84 (A-Form) |
| 52 | 4-Cl | H | H | H | H | H | -CH(OH)- | 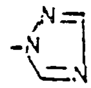 | $-C_5H_{11}$-n | 102 (B-Form) |
| 53 | 4-Cl | H | H | H | H | H | -CH(OH)- | 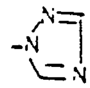 | $-C_3H_7$-i | 161-63 (A-Form) |
| 54 | 4-Cl | H | H | H | H | H | -CH(OH)- | 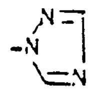 | $-C_3H_7$-i | 74-76 (B-Form) |
| 55 | 4-Cl | H | H | H | H | H | -CH(OH)- | 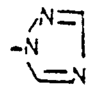 | $-C_4H_9$-i | 110-14 |
| 56 | 4-Cl | H | H | H | H | H | -CH(OH)- | 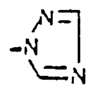 | $-C_4H_9$-i | 107 (A-Form) |
| 57 | 4-Cl | H | H | H | H | H | -CH(OH)- | 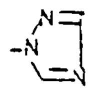 | $-C_4H_9$-i | 151 (B-Form) |
| 58 | 4-Cl | H | H | H | H | H | -CH(OH)- | 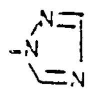 | $-C_3H_7$-i | 160-62 |
| 59 | 4-Cl | H | H | H | H | H | -CH(OH)- | 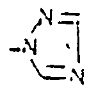 | $-CH_2C{\equiv}CH$ | 90 |
| 60 | 4-Cl | H | H | H | H | H | -CH(OH)- | 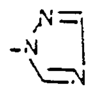 | $-CH_2CH{=}CH_2$ | 107 |
| 61 | H | H | H | H | H | H | -CH(OH)- | 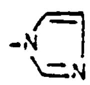 | $-C_3H_7$-n | 1,5600 |
| 62 | H | H | H | H | H | H | -CH(OH)- | 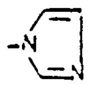 | $-C_3H_7$-i | 128 |
| 63 | 4-Cl | H | H | H | H | H | -CH(OH)- | 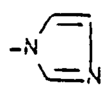 | $-C_2H_5$ | 48-52 |
| 64 | 4-Cl | H | H | H | H | H | -CH(OH)- | 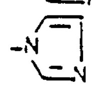 | $-CH_3$ | zähes Oel |
| 65 | 4-Cl | H | H | H | H | H | -CH(OH)- | 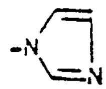 | $-C_4H_9$-n | 120-30 |
| 66 | 4-Cl | H | H | H | H | H | -CH(OH)- | 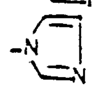 | $-C_3H_7$-i | 50-55 |
| 67 | 4-Cl | H | H | H | H | H | -CH(OH)- | 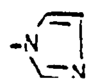 | $-C_3H_7$-n | 44-48 |

A- und B-Form = die beiden möglichen geometrischen Isomeren

Verwendungsbeispiele

In den nachfolgenden Beispielen werden die nachstehend angegebenen Verbindungen als Vergleichssubstanzen eingesetzt.

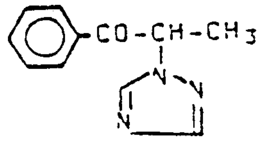

(A)

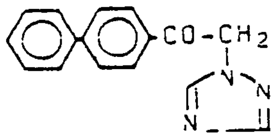

(B)

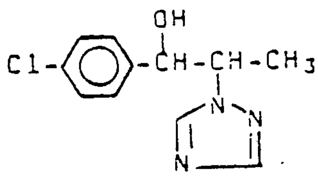

(C)

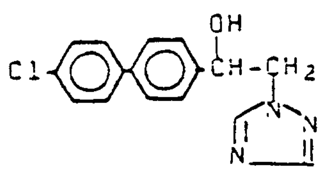

(D)

Beispiel A

Antimykotische in-vitro-Wirksamkeit

Versuchsbeschreibung :

Die in-vitro-Prüfungen wurden im reihenverdünnungstest mit Keiminokula von durchschnittlich $5 \times 10^4$ Keimen/ml Substrat durchgeführt. Als Nährmedium dienten

a) für Dermatophyten und Schimmelpilze : Sabourrand's milieu d'épreuve

b) für Hefen : Fleischextrakt-Traubenzucker-Bouillon.

Die Bebrütungstemperatur betrug 20 °C, die Bebrütungsdauer lag bei 24 bis 96 Stunden bei Hefen und 96 Stunden bei Dermatophyten und Schimmelpilzen.

In diesem Test zeigen insbesondere die Verbindungen der Beispiele 7, 9, 13, 21, 22, 23, 24, 25, 26, 27, 30, 31, 32, 33, 34, 35, 36, 37, 38, 40, 41, 42, 43, 61, 63, 64, 65, 66 und 67 eine bessere antimykotische Wirkung als die aus dem Stand der Technik bekannten Verbindungen (A), (B), (C) und (D).

(Siehe Tabelle A Seite 12 f.)

Tabelle A

| Antimykotische in-vitro-Wirksamkeit | MHK-Werte in γ/ml Nährmedium | | | | |
|---|---|---|---|---|---|
| Wirkstoff | Tricho-phyton mentagr. | Candida albi-cans | Asper-gillus fumig. | Micro-sporum canis | Toru-lopsis glabrata |
| (A) (bekannt) | >64 | >64 | - | - | - |
| (B) (bekannt) | 4 | >64 | >64 | >64 | - |
| (C) (bekannt) | >64 | >64 | - | - | - |
| (D) (bekannt) | 4 | >64 | >64 | >64 | - |
| gemäß Herst.Bsp.Nr. | | | | | |
| 7 | 4 | 16 | 32 | 2 | 64 |
| 9 | 2 | 8 | 8 | 4 | 16 |
| 13 | 4 | 64 | 8 | 8 | - |
| 21 | 4 | 4 | 8 | 4 | 8 |
| 22 | <1 | <1 | <1 | 8 | <1 |
| 23 | 8 | 4 | 16 | - | 16 |
| 24 | <1 | <1 | <1 | <1 | <1 |
| 25 | <1 | <1 | <1 | 2 | 2 |
| 26 | <1 | 8 | 32 | 8 | 8 |
| 27 | <1 | <1 | >6. | 32 | <1 |
| 30 | <1 | <1 | 64 | 8 | <1 |
| 31 | <1 | <1 | 32 | 32 | >64 |
| 32 | <1 | <1 | 32 | 8 | 2 |
| 33 | <1 | 4 | 64 | 32 | 16 |
| 34 | 4 | <1 | 32 | 4 | 2 |
| 35 | <1 | 8 | 32 | 8 | 64 |
| 36 | 4 | <1 | 16 | 16 | 4 |
| 37 | <1 | <1 | >64 | 8 | <1 |
| 38 | 4 | <1 | 32 | 16 | 8 |
| 40 | <1 | <1 | >64 | 16 | 4 |
| 41 | <1 | <1 | >64 | 32 | >64 |
| 42 | 2 | <1 | >64 | 32 | <1 |
| 43 | <1 | <1 | 32 | 8 | 8 |
| 61 | 4 | <1 | 32 | 16 | 32 |
| 63 | 2 | <1 | 16 | 8 | <1 |
| 64 | 8 | <1 | 16 | - | 2 |
| 65 | <1 | <1 | 32 | 8 | 2 |
| 66 | 4 | 4 | 64 | 16 | 2 |
| 67 | <1 | <1 | 64 | 8 | <1 |

## Beispiel B

Antimykotische in-vivo-Wirksamkeit (oral) bei Mäusecandidose

Versuchsbeschreibung :

Mäuse vom Typ SPF-$CF_1$ wurden intravenös mit 1-2 × $10^6$ logarithmisch wachsenden Candida-Zellen, die in physiologischer Kochsalzlösung suspendiert werden, infiziert. Eine Stunde vor und sieben Stunden nach der Infektion werden die Tiere mit jeweils 50-100 mg/kg Körpergewicht der Präparate oral behandelt.

Ergebnis :

Unbehandelte Tiere starben 3 bis 6 Tage post infektionem. Die Ueberlebensrate am 6. Tag post infektionem betrug bei unbehandelten Kontrolltieren etwa 5 %.

In diesem Test zeigten z. B. die erfindungsgemäßen Verbindungen 6, 9, 24, 26, 29, 30, 32, 33, 35, 43, 45, 46, 47, 70 und 71 eine bessere Wirkung als die aus dem Stand der Technik bekannten Verbindungen (A), (B), (C) und (D).

Zeichenerklärung :

+++++ = sehr gute Wirkung = 90 % Ueberlebende am 6. Tag p. i.
++++ = gute Wirkung = 80 % Ueberlebende am 6. Tag p. i.
+++ = Wirkung = 60 % Ueberlebende am 6. Tag p. i.
++ = schwache Wirkung = 40 % Ueberlebende am 6. Tag p. i.
+ = Spur Wirkung =
k.W. = keine Wirkung

### Tabelle B

Antimykotische in-vivo-Wirksamkeit (oral) bei Mäusecandidose

| Wirkstoff | Wirkung |
|---|---|
| (A) (bekannt) | k.W. |
| (B) (bekannt) | k.W. |
| (C) (bekannt) | k.W. |
| (D) (bekannt) | k.W. |
| gemäß Herst. 8 sp. Nr. | |
| 6 | +++ |
| 9 | +++ |
| 21 | +++ |
| 23 | + |
| 26 | +++++ |
| 27 | +++ |
| 29 | +++++ |
| 30 | +++++ |
| 32 | +++ |
| 40 | +++++ |
| 42 | + |
| 43 | ++++ |
| 63 | + |
| 64 | +++ |

## Beispiel C

Antimikrobielle in-vivo-Wirksamkeit (lokal) am Modell der experimentellen Meerschweinchen-Trichophytie

Versuchsbeschreibung :

Weiße Mäuse der Rasse Pirbright-white wurden auf dem geschorenen, nicht skarifizierten Rücken mit einer Mikro- und Makrokonidien-Suspension von Trichophyton mentagrophytes infiziert.

Die infizierten Tiere wurden, beginnend mit dem 3. Tag p. i., 1× täglich mit einer 15 %-igen Lösung der erfindungsgemäßen Präparate (in Dimethylsulfoxid : Glycerin = 1 : 4 = lokal behandelt.

Ergebnis :

Bei unbehandelten Tieren entwickelte sich innerhalb von 12 Tagen p. i. das typische Bild einer Dermatophytose mit Rötung, Schuppung und Haarausfall bis zum totalen Integument-Defekt an der Infektionsstelle.

In diesem Test zeigen z. B. die erfindungsgemäßen Verbindungen 6, 21, 26, 27, 29, 31 und 42 bestimmte Wirkungen.

Tabelle C

Antimykotische in-vivo-Wirksamkeit (lokal) am Modell der experimentellen Meerschweinchen - Trichophytie

| Wirkstoff | Wirkung |
|---|---|
| 6 | ++++ |
| 21 | +++ |
| 26 | +++ |
| 27 | ++ |
| 29 | +++ |
| 32 | +++ |
| 40 | +++ |
| 42 | + |
| 43 | + |

+++++ = sehr gute Wirkung = keine Infektionszeichen am 12.-15. Tag p. i.
++++ = gute Wirkung = geringe Rötung, vereinzelt Schuppen
+++ = Wirkung = Rötung, Schuppung, ohne Haarausfall
++ = schwache Wirkung = Rötung, Schuppung, Haarausfall
+ = Spur Wirkung = flächiger Haarausfall, entzündliche Hautreaktion

Beispiel/Formulierungen

1) Lösung :

| | |
|---|---|
| Wirkstoff gemäß Formel (I) : | 10 g |
| Alkohol, rein (96 %-ig) : | 300 g |
| Isopropylmyristat : | 526 g |
| | 836 g |

2) Creme :

| | |
|---|---|
| Wirkstoff gemäß Formel (I) : | 10 g |
| Arlacel 60 : | 20 g |
| (Sorbitan-monostearat) Tween 60 : | 15 g |
| (Polyoxyethylen (20)-sorbitanmonostearat) | |
| Walrat, künstlich : | 30 g |
| (Mischung von Estern von gesättigten Fettsäuren $C_{14}$-$C_{18}$ und Fettalkoholen $C_{14}$-$C_{18}$ | |
| Lanette 0 : | 100 g |
| (Gemisch aus Cetyl-Alkohol und Stearyl-Alkohol) | |
| Entanol G : | 135 g |
| (2-Octyl-dodecanol) | |
| Benzylalkohol : | 10 g |
| Wasser, entmineralisiert : | 680 g |
| | 1 000 g |

**Patentansprüche**

1. Phenoxyphenyl-azolylmethyl-ketone und -carbinole der allgemeinen Formel

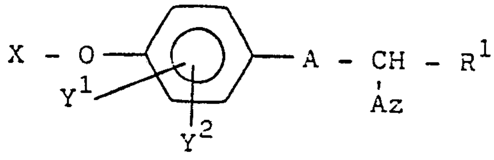

(I)

in welcher

Az für 1,2,4-Triazol-1-yl und -4-yl oder Imidazol-1-yl steht,

A für die Ketogruppe, die —CH(OH)— oder die —C(OH)R-Gruppierung steht,

R für Alkyl mit 1 bis 4, Alkenyl mit 2 bis 4 Kohlenstoffatomen oder gegebenenfalls durch Chlor substituiertes Benzyl steht,

$R^1$ für geradkettiges oder verzweigtes Alkyl mit 1 bis 8 Kohlenstoffatomen, Cycloalkyl und Cycloalkylmethyl mit jeweils 5 oder 6 Kohlenstoffatomen im Cycloalkylteil, Alkenyl mit 2 bis 6 Kohlenstoffatomen, Alkinyl mit 2 bis 4 Kohlenstoffatomen, Alkoxyalkyl und Alkylthioalkyl mit jeweils 1 bis 2 Kohlenstoffatomen in jedem Alkylteil, Halogenalkyl mit 1 bis 4 Kohlenstoff- und 1 bis 5 Halogenatomen, Halogenalkoxyalkyl und Halogenalkylthioalkyl mit jeweils 1 bis 2 Kohlenstoffatomen in jedem Alkylteil und 1 bis 5 Halogenatomen, Dialkylaminoalkyl mit 1 bis 2 Kohlenstoffatomen in jedem Alkylteil, und gegebenenfalls substituiertes Benzyl steht, wobei als Phenylsubstituenten Wasserstoff, Fluor, Chlor, Brom, Methyl, Trifluormethyl, Trifluormethoxy, Trifluormethylthio, Methoxy, Methylthio, Amino, Dimethylamino, Methoxycarbonyl, Nitro oder Cyano in Frage kommen, steht ; und

$Y^1$ für Wasserstoff, Fluor, Chlor oder Methyl steht ;

$Y^2$ für Wasserstoff, Fluor oder Chlor steht ; und

X für gegebenenfalls ein- oder mehrfach durch Chlor oder Methyl oder einfach durch Brom substituiertes Phenyl steht,

sowie deren Säureadditionssalze zur Bekämpfung von Krankheiten.

2. Verbindungen nach Anspruch 1 gekennzeichnet durch die Formel

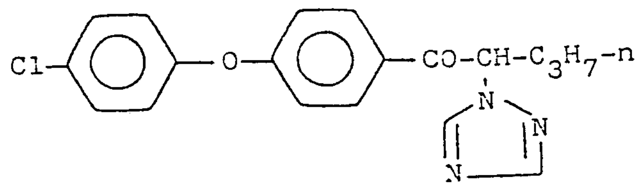

zur Bekämpfung von Krankheiten.

3. Verbindungen nach Anspruch 1 gekennzeichnet durch die Formel

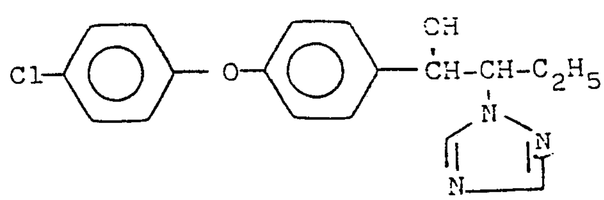

zur Bekämpfung von Krankheiten.

4. Verbindungen nach Anspruch 1 gekennzeichnet durch die Formel

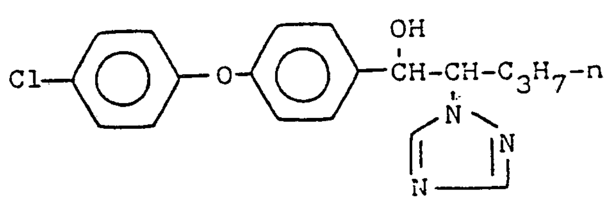

zur Bekämpfung von Krankheiten.

5. Verbindungen nach Anspruch 1 gekennzeichnet durch die Formel

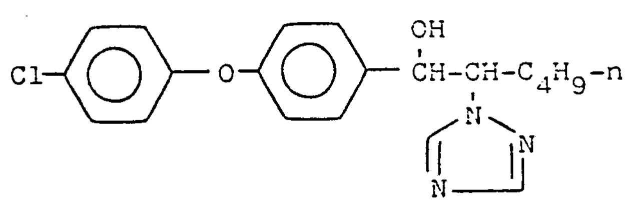

zur Bekämpfung von Krankheiten.

6. Verbindungen nach Anspruch 1 gekennzeichnet durch die Formel

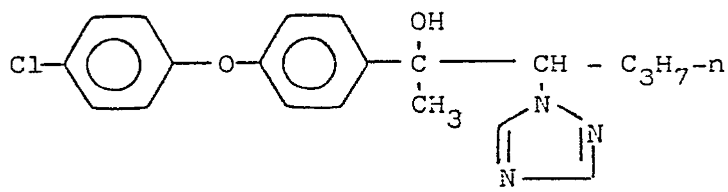

zur Bekämpfung von Krankheiten.

7. Verwendung von Phenoxyphenyl-azolylmethyl-ketonen und -carbinolen der allgemeinen Formel

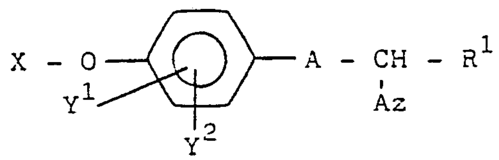

(I)

in welcher

Az für 1,2,4-Triazol-1-yl und -4-yl oder Imidazol-1-yl steht,

A für die Ketogruppe, die —CH(OH)— oder die —C(OH)R-Gruppierung steht,

A für Alkyl mit 1 bis 4, Alkenyl mit 2 bis 4 Kohlenstoffatomen oder gegebenenfalls durch Chlor substituiertes Benzyl steht,

$R^1$ für geradkettiges oder verzweigtes Alkyl mit 1 bis 8 Kohlenstoffatomen, Cycloalkyl und Cycloalkylmethyl mit jeweils 5 oder 6 Kohlenstoffatomen im Cycloalkylteil, Alkenyl mit 2 bis 6 Kohlenstoffatomen, Alkinyl mit 2 bis 4 Kohlenstoffatomen, Alkoxyalkyl und Alkylthioalkyl mit jeweils 1 bis 2 Kohlenstoffatomen in jedem Alkylteil, Halogenalkyl mit 1 bis 4 Kohlenstoff- und 1 bis 5 Halogenatomen, Halogenalkoxyalkyl und Halogenalkylthioalkyl mit jeweils 1 bis 2 Kohlenstoffatomen in jedem Alkylteil und 1 bis 5 Halogenatomen, Dialkylaminoalkyl mit 1 bis 2 Kohlenstoffatomen in jedem Alkylteil, und gegebenenfalls substituiertes Benzyl steht, wobei als Phenyl substituenten Wasserstoff, Fluor, Chlor, Brom, Methyl, Trifluormethyl, Trifluormethoxy, Trifluormethylthio, Methoxy, Methylthio, Amino, Dimethylamino, Methoxycarbonyl, Nitro oder Cyano in Frage kommen, steht ; und

$Y^1$ für Wasserstoff, Fluor, Chlor oder Methyl steht ;

$Y^2$ für Wasserstoff, Fluor oder Chlor steht ; und

X für gegebenenfalls ein- oder mehrfach durch Chlor oder Methyl oder einfach durch Brom substituiertes Phenyl steht ; sowie deren Säureadditionssalzen

zur Herstellung von antimykotischen Mitteln.

8. Verbindungen gemäß Ansprüchen 1 bis 6 zur Bekämpfung von Krankheiten.

9. Pharmazeutische Mittel gekennzeichnet durch den Gehalt an mindestens einer des Verbindungen gemäß Ansprüchen 1 bis 7.

**Claims**

1. Phenoxyphenyl azolylmethyl ketones and carbinols of the general formula

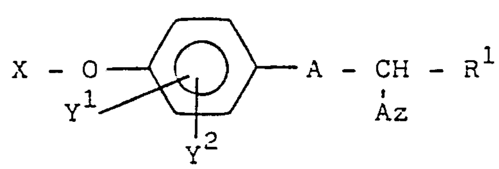

(I)

in which

Az represents 1,2,4-triazol-1-yl and -4-yl or imidazol-1-yl,

A represents the keto group, the —CH(OH) or the —C(OH)R grouping,

R represents alkyl with 1 to 4, alkenyl with 2 to 4 carbon atoms or optinally chlorine-substituted benzyl,

$R^1$ represents straight-chain or branched alkyl with 1 to 8 carbon atoms, cycloalkyl and cycloalkylmethyl with in each case 5 or 6 carbon atoms in the cycloalkyl part, alkenyl with 2 to 6 carbon atoms, alkinyl with 2 to 4 carbon atoms, alkoxyalkyl and alkylthioalkyl with in each case 1 to 2 carbon atoms in each alkyl part, halogenoalkyl with 1 to 4 carbon and 1 to 5 halogen atoms, halogenoalkoxyalkyl and halogenoalkylthioalkyl with in each case 1 to 2 carbon atoms in each alkyl part and 1 to 5 halogen atoms, dialkylaminoalkyl with 1 to 2 carbon atoms in each alkyl part, and optionally substituted benzyl, possible phenyl substituents being hydrogen, fluorine, chlorine, bromine, methyl, trifluoromethyl, trifluoromethoxy, trifluoromethylthio, methoxy, methylthio, amino, dimethylamino, methoxycarbonyl, nitro or cyano ; and

$Y^1$ represents hydrogen, fluorine, chlorine or methyl ;

$Y^2$ represents hydrogen, fluorine or chlorine ; and

X represents phenyl which is optionally mono- or polysubstituted by chlorine or methyl or monosubstituted by bromine,

and acid addition salts thereof, for combating diseases.

2. Compounds according to Claim 1, characterised by the formula

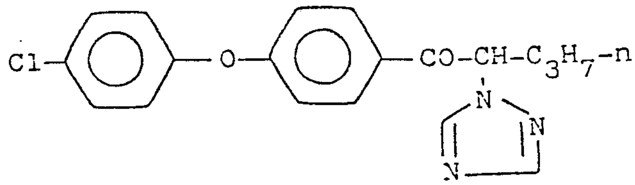

for combating diseases.

3. Compounds according to Claim 1, characterised by the formula

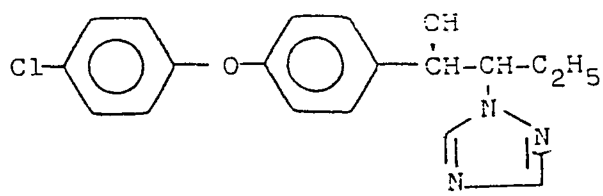

for combating diseases.

4. Compounds according to Claim 1, characterised by the formula

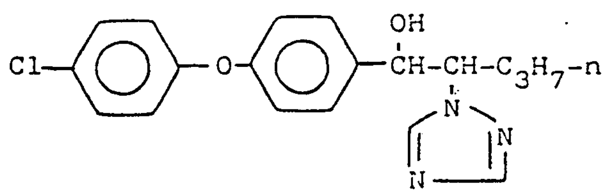

for combating diseases.

5. Compounds according to Claim 1, characterised by the formula

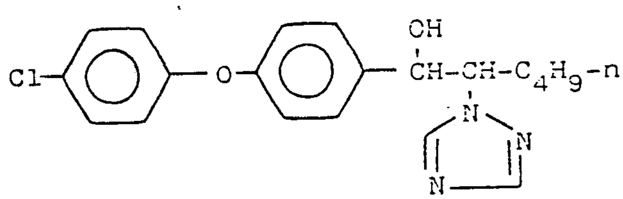

for combating diseases.

6. Compounds according to Claim 1, characterised by the formula

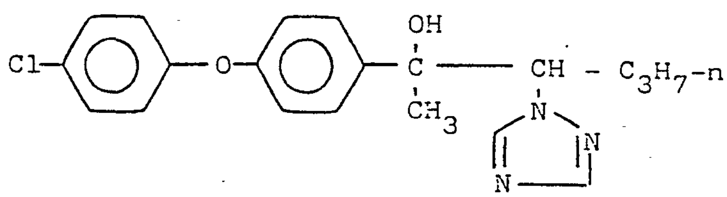

for combating diseases.

7. Use of phenoxyphenyl azolylmethyl ketones and carbinols of the general formula

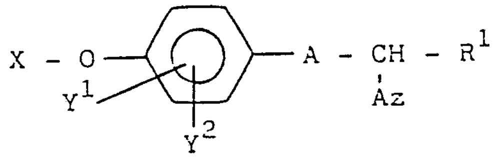

(I)

in which

Az represents 1,2,4-triazol-1-yl and -4-yl or imidazol-1-yl,

A represents the keto group, the —CH(OH) or the —C(OH)R grouping,

R represents alkyl with 1 to 4, alkenyl with 2 to 4 carbon atoms or optionally chlorine-substituted benzyl,

$R^1$ represents straight-chain or branched alkyl with 1 to 8 carbon atoms, cycloalkyl and cycloalkylmethyl with in each case 5 or 6 carbon atoms in the cycloalkyl part, alkenyl with 2 to 6 carbon atoms, alkynyl with 2 to 4 carbon atoms, alkoxyalkyl and alkylthioalkyl with in each case 1 to 2 carbon atoms in each alkyl part, halogenoalkyl with 1 to 4 carbon and 1 to 5 halogen atoms, halogenoalkoxyalkyl and halogenoalkylthioalkyl with in each case 1 to 2 carbon atoms in each alkyl part and 1 to 5 halogen atoms, dialkylaminoalkyl with 1 to 2 carbon atoms in each alkyl part, and optionally substituted benzyl, possible phenyl substitutents being hydrogen, fluorine, chlorine, bromine, methyl, trifluoromethyl, trifluoromethoxy, trifluoromethylthio, methoxy, methylthio, amino, dimethylamino, methoxycarbonyl, nitro or cyano ; and

$Y^1$ represents hydrogen, fluorine, chlorine or methyl ;

$Y^2$ represents hydrogen, fluorine or chlorine ; and

X represents phenyl which is optionally mono- or polysubstituted by chlorine or methyl or monosubstituted by bromine,

and acid addition salts thereof, for the preparation of antimycotic agents.

8. Compounds according to Claims 1 to 6 combating diseases.

9. Pharmaceutical agents, characterised in that they contain at least one of the compounds according to Claims 1 to 7.

**Revendications**

1. Phénoxyphényl-azolylméthyl-cétones et phénoxyphényl-azolylméthyl-carbinols de formule générale :

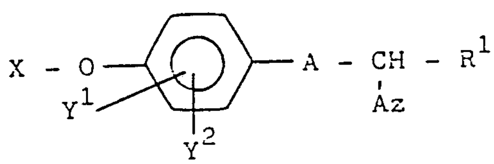

(I)

dans laquelle

Az représente un groupe 1,2,4-triazol-1-yle, un groupe 1,2,4-triazol-4-yle ou un groupe imidazol-1-yle,

A représente le groupe céto, le groupement —CH(OH)— ou le groupement —C(OH)R—,

R représente un groupe alkyle contenant 1 à 4 atomes de carbone, un groupe alcényle contenant 2 à 4 atomes de carbone ou un groupe benzyle éventuellement substitué par un atome de chlore,

$R^1$ représente un groupe alkyle à chaîne droite ou ramifiée contenant 1 à 8 atomes de carbone, un groupe cycloalkyle et un groupe cycloalkylméthyle contenant chacun 5 ou 6 atomes de carbone dans la fraction cycloalkyle, un groupe alcényle contenant 2 à 6 atomes de carbone, un groupe alcynyle contenant 2 à 4 atomes de carbone, un groupe alcoxyalkyle et un groupe alkylthioalkyle contenant chacun 1 ou 2 atomes de carbone dans chaque fraction alkyle, un groupe halogénalkyle contenant 1 à 4 atomes de carbone et 1 à 5 atomes d'halogènes, un groupe halogénalcoxyalkyle et un groupe halogénalkylthioalkyle contenant chacun 1 ou 2 atomes de carbone dans chaque fraction alkyle et 1 à 5 atomes d'halogènes, un groupe dialkylaminoalkyle contenant 1 ou 2 atomes de carbone dans chaque fraction alkyle, ainsi qu'un groupe benzyle éventuellement substitué, en envisageant, comme substituants du groupe phényle, l'hydrogène, le fluor, le chlore, le brome, le groupe méthyle, le groupe trifluorométhyle, le groupe trifluorométhoxy, le groupe trifluorométhylthio, le groupe méthoxy, le groupe méthylthio, le groupe amino, le groupe diméthylamino, le groupe méthoxycarbonyle, le groupe nitro ou le groupe cyano ; et

$Y^1$ représente l'hydrogène, le fluor, le chlore ou le groupe méthyle ;

$Y^2$ représente l'hydrogène, le fluor ou le chlore ; et

X représente un groupe phényle éventuellement substitué une ou plusieurs fois par un atome de chlore ou par un groupe méthyle ou une fois par un atome de brome,

ainsi que leurs sels d'addition d'acide en vue de combattre des maladies.

2. Composés selon la revendication 1, caractérisés en ce qu'ils répondent à la formule :

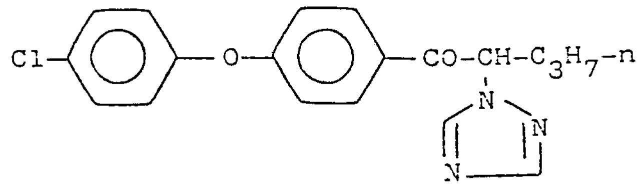

en vue de combattre des maladies.

3. Composés selon la revendication 1, caractérisés en ce qu'ils répondent à la formule :

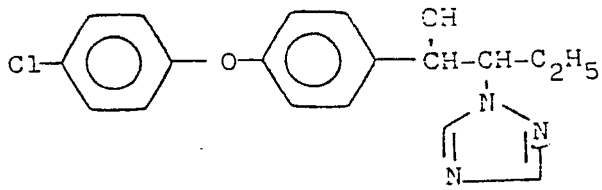

en vue de combattre des maladies.

4. Composés selon la revendication 1, caractérisés en ce qu'ils répondent à la formule :

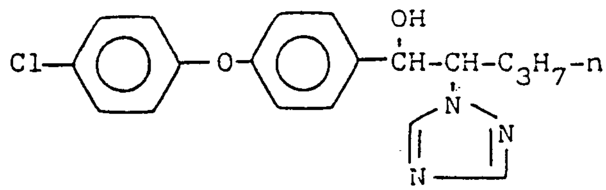

en vue de combattre des maladies.

5. Composés selon la revendication 1, caractérisés en ce qu'ils répondent à la formule :

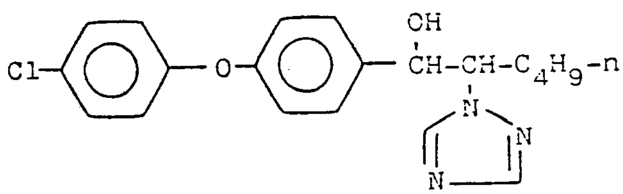

en vue de combattre des maladies.

6. Composés selon la revendication 1, caractérisés en ce qu'ils répondent à la formule :

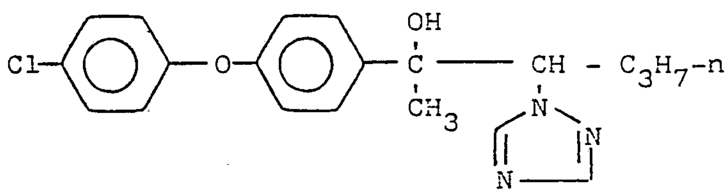

en vue de combattre des maladies.

7. Utilisation de phénoxyphényl-azolyl-méthyl-cétones et de phénoxyphényl-azolylméthyl-carbinols de formule générale :

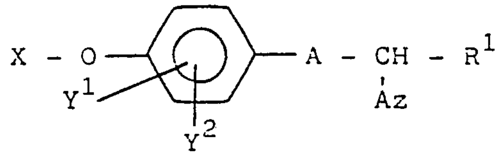

(I)

dans laquelle

Az représente un groupe 1,2,4-triazol-1-yle, un groupe 1,2,4-triazol-4-yle ou un groupe imidazol-1-yle,
A représente le groupe céto, le groupement —CH(OH)— ou le groupement —C(OH)R—,

R représente un groupe alkyle contenant 1 à 4 atomes de carbone, un groupe alcényle contenant 2 à 4 atomes de carbone ou un groupe benzyle éventuellement substitué par un atome de chlore,

$R^1$ représente un groupe alkyle à chaîne droite ou ramifiée contenant 1 à 8 atomes de carbone, un groupe cycloalkyle et un groupe cycloalkylméthyle contenant chacun 5 ou 6 atomes de carbone dans la fraction cycloalkyle, un groupe alcényle contenant 2 à 6 atomes de carbone, un groupe alcynyle contenant 2 à 4 atomes de carbone, un groupe alcoxyalkyle et un groupe alkylthioalkyle contenant chacun 1 ou 2 atomes de carbone dans chaque fraction alkyle, un groupe halogénalkyle contenant 1 à 4 atomes de carbone et 1 à 5 atomes d'halogènes, un groupe halogénalcoxyalkyle et un groupe halogénalkylthialkyle contenant chacun 1 ou 2 atomes de carbone dans chaque fraction alkyle et 1 à 5 atomes d'halogènes, un groupe dialkylaminoalkyle contenant 1 ou 2 atomes de carbone dans chaque fraction alkyle, ainsi qu'un groupe benzyle éventuellement substitué, en envisageant, comme substituants du groupe phényle, l'hydrogène, le fluor, le chlore, le brome, le groupe méthyle, le groupe trifluorométhyle, le groupe trifluorométhoxy, le groupe trifluorométhylthio, le groupe méthoxy, le groupe méthylthio, le groupe amino, le groupe diméthylamino, le groupe méthoxycarbonyle, le groupe nitro ou le groupe cyano ; et

$Y^1$ représente l'hydrogène, le fluor, le chlore ou le groupe méthyle ;

$Y^2$ représente l'hydrogène, le fluor ou le chlore ; et

X représente un groupe phényle éventuellement substitué une ou plusieurs fois par le chlore ou le groupe méthyle ou une fois par le brome ;

ainsi que leurs sels d'addition d'acide pour préparer des agents antimycotiques.

8. Composés selon les revendications 1 à 6, en vue de combattre des maladies.

9. Agents pharmaceutiques, caractérisés en ce qu'ils contiennent au moins un des composés selon les revendications 1 à 7.